# EUROPEAN PATENT SPECIFICATION

(11) **EP 1 520 579 B1**
(45) Date of publication and mention of the grant of the patent: **25.03.2009**
(21) Application number: 04023414.8
(22) Date of filing: 01.10.2004
(51) Int. Cl.: A61K 31/095, A61K 9/70, A61K 47/10, A61K 47/12, A61K 47/44, A61P 35/00

(54) **Compositions for the treatment and prevention of cancer**
Arzneimittel zur Behandlung und zur Prävention von Krebs
Compositions pharmaceutiques pour le traitement et la prévention du cancer

(30) Priority: 01.10.2003 WO PCT/EP03/10880
(43) Date of publication of application: 06.04.2005
(73) Proprietor: Schrauzer, Gerhard N., Coronado CA 92118 (US)
(72) Inventor: Schrauzer, Gerhard N., Coronado CA 92118 (US)
(74) Representative: Grünecker, Kinkeldey, Stockmair & Schwanhäusser Anwaltssozietät

(56) References cited:
- EP-A- 1 205 471
- US-A1- 2002 197 304
- HOWARD E. GANTHER: "Selenium metabolism, selenoproteins and mechanisms of cancer prevention: complexities with thioredoxin reductase" CARCINOGENESIS, vol. 20, no. 9, 1999, pages 1657-1666, XP002312585
- P.A.C. 'T HOEN ET ALL.: "Induction of glutathione-S-transferase mRNA levels by chemoprventive selenocysteine Se-conjugates" BIOCHEMICAL PHARMACOLOGY, vol. 63, 2002, pages 1843-1849, XP002312586
- IP C ET AL: "RELATIONSHIP BETWEEN THE CHEMICAL FORM OF SELENIUM AND ANTICARCINOGENIC ACTIVITY" CANCER CHEMOPREVENTION, XX, XX, August 1992 (1992-08), pages 479-488, XP000926082
- GERALD F. COMBS ET ALL.: "Chemoprotective agents: Selenium" PHARMACOL. THER, vol. 79, no. 3, 1998, pages 179-192, XP002312587
- TAPIERO H ET AL: "THE ANTIOXIDANT ROLE OF SELENIUM AND SELENO-COMPOUNDS" BIOMEDICINE AND PHARMACOTHERAPY, ELSEVIER, PARIS, FR, vol. 57, no. 3/4, 2003, pages 134-144, XP001202166 ISSN: 0753-3322

## Description

### Background of the Invention

Selenium is an essential trace element and is the functional component of several important enzymes and which is increasingly also recognized for its anticarcinogenic properties. The selenium-dependent glutathione peroxidases, for example, prevent the build-up of hydrogen peroxide and lipid hydroperoxides and thus help to protect cells and tissues from damage by reactive oxygen species. Since oxygen radicals attacking DNA may cause mutations resulting in the transformation of normal cells into cancer cells, selenium by virtue of its antioxidant properties can counteract these processes thereby acting as a cancer-protecting agent.

Selenium in addition to its anti-mutagenic properties may alter carcinogen metabolism and prevent DNA methylation and other mechanism believed to play a role in carcinogenesis. Selenium is also required for the maintenance of the functions of the immune system. At pharmacological levels, selenium is known to exert cytotoxic effects on tumor cells without adversely affecting normal cells. For example, sodium selenite added to the growth media caused the selective destruction of human hepatoma and lung cancer cells without affecting normal embryonic liver or lung cells; see: S.Y. Yu, et al., Biol. Trace El. Res. 15:243-255 (1987); A. Pung et al., Biol. Trace El. Res. 14:29-42 (1987). Selenium compounds accordingly are receiving attention as potential cancer therapeutic agents. Selenium preparations for a variety of therapeutic uses are already available, most of which are for oral administration or by injection.

A number of selenium-containing preparations for topical applications have also been claimed. For example, in U.S. Patent 4,865,840 the use of lotions or creams containing selenomethionine was claimed for the prevention of ultraviolet radiation-induced skin damage. In U.S. Patent 4,512,977, selenium compounds are claimed for the topical treatment of damage caused by surgical incisions, lacerations and burns. The compounds claimed in these patents are water-soluble.

A lipid soluble organoselenium compound, di-n-hexyl diselenide, has previously been used in cancer therapy, but only in preparations for oral or parenteral administration (E. Revici, Research in Physiopathology as Basis of Guided Chemotherapy, Van Nostrand Company, New York, 1961, pp. 512-518). The related compound di-n-diheptyl diselenide was shown to inhibit the growth of several human cancer cell lines in cell cultures [see G. Scambia et al., Anticancer Res. 9: 1697-1700, (1989). In German Patent 43 20694.8-41, preparations containing dialkyl diselenides were claimed for the topical treatment of warts. For cancer treatment, preparations containing dialkyl diselenides and related compounds have previously been claimed only for oral or parenteral administration. For example, in EP-0095663, the reaction product of selenium with eleostearic acid was claimed as an anti-neoplastic drug for oral or parenteral administration. In DE-3408362 C2, dialkyl diselenides of the general formula R-Se-Se-R, with R being an alkyl or alkenyl group with an odd number of carbon atoms, in combination with an aliphatic ketone were claimed as oral and parenteral drugs for the treatment of cancer. DE-3408362 refers to adverse side effects of said dialkyl diselenides, which are reduced but not diminished by administering the active selenium compound combined with the aliphatic ketone.

Thus, there is still a need in the art for means to treat cancer and to improve existing treatment regimen and to overcome the problems of toxicity associated with the prior art administration of such compounds even in high dosages.

The present invention is based on surprisingly positive clinical results showing that organoselenium components and, in particular selenols as well as related compounds, can be used very conveniently and effectively with low adverse side effects for the topical and transdermal treatment and/or prevention of cancer in organs such as the skin, the breast, the rectum, and the large intestine as well as for the intratumoral or peritumoral injection for the treatment and/or prevention of said cancer.

Consequently, the above-identified problems of obtaining means for treating cancers showing a decreased toxicity are solved in accordance with the present invention by the use of organoselenium compounds as defined in claim 1 for the manufacture of a medicament for topical treatment and/or prevention of cutaneous and subcutaneous cancer and pre-cancerous conditions.

### Summary of the Invention

The present invention provides the use of lipid soluble organoselenium compounds for the manufacture of medicaments for topical application in humans and animals. The topical organoselenium preparations may be used for the prevention and/or treatment of cutaneous and subcutaneous cancer and pre-cancerous conditions in organs such as the skin, the breast, the rectum, and the large intestine.

Suitable lipid soluble organoselenium compounds are of the general formula

R₁-(Se)ₙ-R₂

in which n is an integer from 1 to 8, and R₁ and R₂ are the same or different and each represents H or an aralkyl, cycloalkyl, aryl or any alkyl group having from 1 to 50, preferably 5 to 12 carbon atoms, wherein the terminal carbon atom of each of said alkyl groups may be substituted with a carboxyl group, with the proviso that at least one of R₁ and R₂ is not H.

According to current knowledge, cancer cells develop from normal cells in a stepwise process involving several mutations and other irreversible genetic changes. The process may be triggered by a wide variety of agents and stress factors, including high-energy radiation, viruses, chronic irritants, toxic heavy metals, certain chemicals and drugs. Cells after one mutational event may still be essentially benign but may subsequently undergo further changes and become malignant. Malignant cells are usually recognized as foreign by the cells of the immune system and are destroyed. However, cells evading the immunological surveillance system will continue to proliferate and accumulate, initially forming a small, avascular tumor. This tumor will grow slowly until it develops its own blood supply, at which point growth rates usually increase, and sometimes dramatically.

Because of the multistage nature of the process of carcinogenesis, the term "pre-cancerous conditions" was used in the present patent disclosure to denote all states of cells with the potential of becoming malignant, irrespective of the nature of the carcinogenic agent.

The present invention provides compositions for cancer treatment and prevention that can be applied in the earliest as well as in more advanced stages of tumor development. The compositions contain members of a group of relatively nontoxic lipid soluble organoselenium compounds as defined above and inert carriers to facilitate their transport through the skin. Treatment of a tumor is accomplished by applying these compositions directly onto the tumor or on the skin above and around it. The organoselenium compounds may be applied in the form of lotions, oils, creams, sprays or suppositories (via mucous tissues similar to dermal tissue). In addition, they may be dissolved in polymeric matrices to be used as transdermal patches for their slow release into affected areas.

Pharmaceutical compositions suitable for topical treatment and/or prevention of cutaneous and subcutaneous cancer and pre-cancerous conditions according to the present invention are solutions or dispersions of one or more of said lipophilic organoselenium compounds in a physiologically acceptable carrier capable of absorption through skin, wherein the amount of said organoselenium compound(s) is therapeutically effective. Depending on their intended use, the concentration of the organoselenium compounds in the different compositions may range from 0.01 to 40 %. The preferred concentration ranges upon application of the composition onto skin are in the range of from 0.1 % to 21 %. A specific range is found to be from 6 to 12% in an aliphatic hydrocarbon or from 1 to 21 %, preferably 19 to 21 %, in creams and oils. The concentration in a spray composition where some of the carrier evaporates upon application to skin may be as low as 0.01 %.

### Preferred embodiment of the invention

The present application provides in a preferred embodiment the use of alkyl selenols of the general formula RSeH, wherein R is an alkyl, aryl, aralkyl or cycloalkyl residue of 1 - 50 carbon atoms as such or in suitable inert liquid carrier solvents, solid or semisolid matrices, or gaseous form carriers for topical, and transdermal applications, as well as for intratumoral or peritumoral injections for the manufacture of a medicament for the treatment and prevention of cancer. The present invention claims the use of selenols for a new purpose which has not been described before. This includes US Patent application No. 20020197304A1 of Dec. *26*,*2002*, wherein alkyl selenols were mentioned, but only dialkyl diselenides, RSeSeR, were mentioned as cancer therapeutic agents. In the general formula on page 3 above, preferred integers are n=1 and 3 to 8.

It has now been surprisingly found that alkyl selenols are more potent antitumoral agents than the corresponding dialkyl diselenides, especially if applied under conditions preventing their oxidation to diselenides. As alkyl selenols are sensitive to oxidation, they are preferably applied under exclusion of oxygen. The alkyl selenols also have pungent odors. The present application therefore also claims therapeutic pads allowing the application of selenols under exclusion of air and with minimal release of the compounds into the surrounding airspace.

Without being bound to any theory, it is disclosed that the therapeutic efficacy of the alkylselenols depends on (1) the length of the alkyl residue R, and (2) on the composition of the inert carriers. The alkyl chain length must be selected to provide a selenol of sufficiently low vapor pressure on the one hand and the ability to penetrate through the skin on the other hand. It was found that n-hexyl selenol, n-heptyl selenol and n-octyl selenol are most suitable. Alkyl selenols with alkyl chain lengths shorter than 6 must be carefully handled for the intended application due to their increasingly higher vapor pressures. The alkyl chain length also affects the systemic toxicity of the selenols. For example, n-Hexyl selenol is less toxic than methyl selenol, CH₃-SeH. The latter furthermore is gaseous at room temperature and its extremely pungent odor precludes its practical applications.

Also structural requirements for the carrier should be considered to provide a formulation which best serve the needs of the patent: It should combine a sufficiently low vapor pressure to prevent its evaporation on the skin and still have the ability to penetrate through the skin. Whereas n-decane, C₁₀H₂₄ has excellent penetrating properties, n-dodecane, C₁₂H₂₆, is already less penetrating but still suitable. Further increases of alkyl chain lengths ultimately result in little or even no skin permeation. Mixtures of n-decane with 1-30 weight-percent of higher n-alkanes may be used to reduce possible skin irritation or defattening action of n-decane of patients with sensitive skin.

Additions of certain vegetable oils such as canola oil, olive oil, jojoba oil, or sesame oil can be used in cases of sensitivity to the paraffinic hydrocarbon carriers, at levels ranging from 1-99 wt%. Preferred compositions for the use according to the invention are solutions containing 0,1 - 21 wt.-% of n-hexylselenol in n-decane containing 1- 20 wt % of a mixture of higher n-alkanes, e.g. as present in "light petrolatum", a common and widely used emulent, or some of the above mentioned vegetable oils.

### Detailed description of the invention

In a preferred embodiment of the present application, the specific use of selenols (RSeH) as active cancer therapeutic agents is claimed. This new teaching is based on a new finding that alkyl selenols, when applied under conditions preventing their oxidation, are highly effective in destroying tumors. The present application thus is fundamentally new. The therapeutic efficacy and applicability of alkyl selenols depends on the length of the alkyl chain R, as it determines the physical properties of the selenols and, what is most important for their present application, ability to penetrate through the skin. In this context, it was found that selenols carrying a linear alkyl chain R composed of 5 - 12 carbon atoms are best suited. Selenols (RSeH) with alkyl chains of fewer than 5 carbon atoms are often too volatile to be used successfully, whereas selenols with alkyl chains exceeding 12 carbon atoms still pass through the skin, even to a lesser extent, but will show therapeutic efficacy but are less preferred than selenols with alkyl chains composed of 5 to 12 carbon atoms.

Among the last-mentioned compounds the selenol with R = n-hexyl with structural formula CH₃(CH₂)₄CH₂SeH, is preferred for the treatment of superficial tumors of the skin as well as for deep-seated tumors such as, for example, tumors of the breast. Secondary selenols such as 2-n-hexyl selenol, CH₃-CH(SeH)CH₂-CH₂CH₂CH₃ or 3-n-hexyl selenol CH₃CH₂CH(SeH)CH₂CH₂CH₃ or mixtures thereof in particular together with primary selenols can also be used. Selenols with branched alkyl substituents, for example 2-methylpentylselenol, (CH₃)₂CHCH₂CH₂CH₂SeH likewise are somewhat less efficient as it penetrates skin less well than the linear n-hexylselenol. The same is true for cycloalkyl selenols such as cyclohexylselenol, C₆H₁₁-SeH, cycloheptylselenol, C₇H₁₃SeH, and so forth. Within the general scope of the present application are also aralkyl selenols such as 1-phenylethylselenol, HSeCH₂CH₂C₆H₅. The selenols with branched alkyl chain rapidly oxidize what generally results in diminished skin permeability. The same is true when the n-alkyl residue is replaced by a cycloalkyl group or for aryl selenols, such as C₆H₅SeH; although these compounds all show some effect as active agent they are less efficient when compared to the n-alkylselenols.

Polyselonols, such as diselenols represented e.g. by 1,4-diselenolobutane, HSeCH₂CH₂CH₂CH₂SeH and higher homologs are included in the use of the present invention, the same is true for alkyl, cycloalkyl or aryl tri- and other polyselenols. In polyselenols of the invention 2 to 20 SeH moieties are comprised in an alkyl residue or any molecule, preferably comprising 1 to 50, more preferably 5 to 12 carbon atoms.

The n-alkylselenols or polyselenols are not normally applied as such but rather in combination with a suitable inert transporting agent or a mixture of transporting agents. Preferred transporting agents are linear chain hydrocarbons, preferably with 9-12 carbon atoms, e.g. n-decane, C₁₀H₂₂. n-Decane is preferred because of its superior skin penetrating properties. To the carrier n-decane may be added certain higher molecular weight alkanes as present in light petrolatum, e.g. at concentrations ranging from 0.01 - 40 wt. %, with 19 - 21% being most preferred. Mixtures of n-decane with vegetal oils such as canola oil, olive oil, jojoba oil or sesame oil can also be used, as well as squalene and mixtures thereof.

It is preferable to prepare mixtures of the selenols with the last mentioned additives immediately prior to their application because reactions of the selenol with components in the vegetal oils may occur on storage of such mixtures.

The purpose of these additives is to prevent possible skin irritation that patients with sensitive skin may experience and to influence the depth of skin penetration. For superficial tumors it is advantageous to accumulate as much of the selenol in, and in the vicinity of, the tumor rather than to have it transported from a more distinct application site.

The pure selenol is preferably applied topically and only on small isolated lesions. For larger superficial or subcutaneous tumorous lesions, typically of 1-5 cm diameter, the selenol, such as n-hexylselenol, is applied in dilute form along with the inert carrier. The concentrations of selenol in the carrier may range from 0.01 - 90 wt %, 0.1 - 21 and 19 - 21 wt % being preferred ranges.

For prophylactic purposes, such as protecting the unaffected breast of a breast cancer patient, or for patient maintenance lower concentrations such as 0.1 to 0.5 wt %, are typically but not exclusively used.

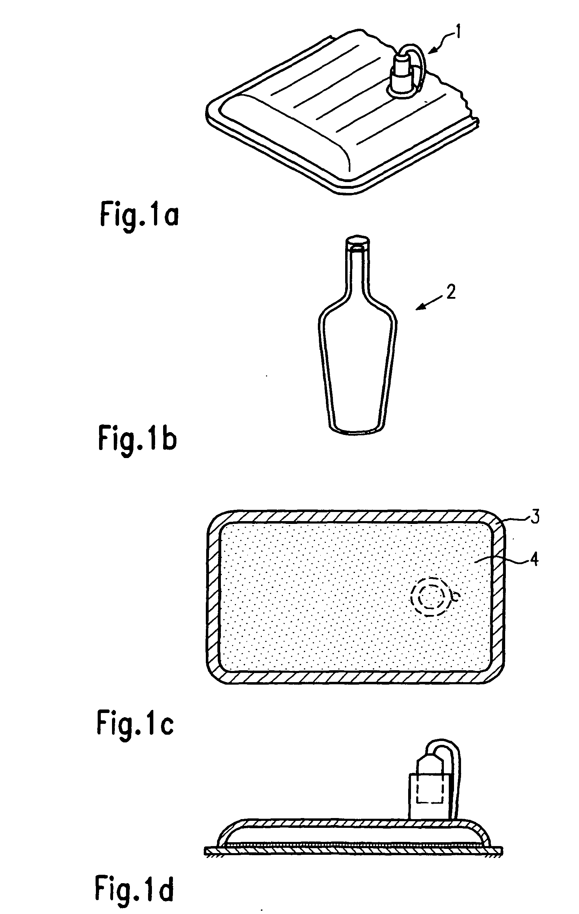

In order to utilize the selenols therapeutically their oxidation during the application must be avoided. In addition, patients and clinical personnel must be protected as much as possible from the pungent odor characteristic of selenols. The area to be treated is first covered with plastic pad of appropriate size, which may have a medical adhesive, attached to it tight-fit. It is preferred to have on the upper side of the pad an opening with a plug for insertion of e.g. a plastic ampoule containing the selenol-carrier mixture (see Figure 1 below). The capacity of the ampoule may e.g. range from 0.5 to 5 cm3.

Organoselenium compounds used in the present invention are comprised in pharmaceutical compositions.

The therapeutic efficacy of the organoselenium compounds as defined above in such pharmaceutical compositions depends on (1) the type and length of the alkyl residues R₁ and R₂, and (2) the composition of the inert carrier as these determine the degree to which they can penetrate through the skin. Preferably, the alkyl residues are unbranched. It is also preferred that the average chain length of said R₁ and R₂ alkyl groups is in the range of from 6-8 carbon atoms. Increasing the average alkyl chain length beyond n-octyl (e.g. in n-octylselenol) causes the pharmaceutical compositions to become less skin penetrating and more suitable for the treatment of superficial cancerous lesions. Shortening the average chain length below n-hexyl increases the volatility of these compounds. This reduces their therapeutic efficacy as it causes them to evaporate when brought onto the skin. As indicated by the term "average chain length", the alkyl residues of said dialkyl organoselenium compounds need not be equal. Thus, e.g. two carbon chains of a length of 5 and 7 carbon atoms, respectively, on an organoselenium compound are regarded as equivalent to one organoselenium compound with 6 carbon atoms. Other equivalencies having suitable volatility and skin absorption properties will be apparent.

For example, the composition may contain an alkyl selenol of the general formula R-SeH, with the chain length of the alkyl group R ranging from 5 to 12 carbon atoms, preferably from 6 to 8 carbon atoms (or, for example, from 5 to 9 where the R residues are not equal). A preferred precursor is n-hexyl selenol. A drawback to be avoided in the administration of the selenols is their unpleasant odor.

Passage of the organoselenium compounds through the skin is facilitated by transporting agents as carriers in the composition. Successful carriers comprise aliphatic hydrocarbons, preferably liquid aliphatic hydrocarbons with 10 to 12 carbon atoms. Specific examples include, n-decane, n-undecane and n-dodecane, where the prefix n-denotes that the carbon chain is linear and unbranched. For example, a lotion containing di-n-hexyl diselenide or n-hexyl selenol in n-decane shows excellent skin penetration properties. Branched hydrocarbons and other carriers that tend to plug pores and thereby impede transfer through skin are generally undesirable.

In a preferred embodiment of the present invention, the pharmaceutical composition comprises 0.1 - 21 %, n-hexyl selenol, or n-heptyl selenol or mixtures thereof in n-decane, n-undecane, n-dodecane or mixtures thereof.

Other suitable carriers include sesame oil, safflower oil, wheat germ oil, sun flower oil, avocado oil, jojoba oil, olive oil, squalane, stearic acid, cetyl alcohol, glycerol, triethanolamine, retinyl palmitate, allantoin, methyl paraben, imidazolidinyl urea, fragrant plant oils, DL-α-tocopherol, DL-α-tocopheryl acetate, vitamin A, and light petrolatum, and other commonly used base materials as equivalents. The carrier may effectively be inert or may contain other therapeutic agents compatible with the organoselenium compound.

Pharmaceutical compositions suitable for application in the form of lotions, oils, creams or sprays may contain the active organoselenium compound(s) and additional ingredients such as aliphatic hydrocarbons, preferably with 10 to 12 carbon atoms, fats or oils of plant, animal or synthetic origin, emulsified fat- other water-soluble vitamins, mineral oils, hormones, prohormones, antihormones, antineoplastic drugs, fragrances and analgesic substances, organ extracts, plant extracts, synthetic or natural polymeric substances, along with water and emulsifiers. Preferably, these compositions comprise the active organoselenium compound(s) in an amount of from 0.01 - 40 %, more preferably 0,1-21 %, per weight.

If the organoselenium compounds are rubbed onto the skin in the form of lotions, creams or oils, locally high concentrations of the organoselenium species can be generated in organs at risk of developing cancer. The preferred active ingredients in these compositions for extemal cancer treatment are alkyl selenols as defined above with the average chain length of the alkyl residues preferably in the range from 6 to 8 carbon atoms.

In particular, when they are to be applied in rubbing oils for preventive rather than therapeutic purposes, base materials in common usage such as sesame oil, safflower oil, wheat germ oil, squalane, stearic acid, cetyl alcohol, glycerol, triethanol amine, retinyl palmitate, allantoin, methyl paraben, imidazolidinyl urea, and fragrant oils may be used. These ingredients along with emulsifying agents and water are typically used in creams containing the organoselenium compounds. Other ingredients include DL-α-tocopherol (vitamin E), DL-α-tocopheryl acetate (vitamin E acetate) or vitamin A. Other materials on the GRAS list, or otherwise known to be suitable for application to skin are equivalent.

In a preferred embodiment of the present invention, alkyl selenols are added to vegetable oils such as safflower oil, sunflower oil, sesame oil, avocado oil, jojoba oil, wheat germ oil, olive oil, and fragrant oils in an amount of from 0,01 - 40 % by weight, preferable from 0,1 - 21 % by weight.

The organoselenium compounds may also be applied in the form of suppositories into body cavities, using low-melting natural or synthetic lipophilic substances such as cocoa butter or polyethylene oxide, polyethylene glycol or polypropylene glycol as inert carriers. Other materials suitable for suppositories may be used as well. Preferably, the low-melting substances or mixtures thereof have melting points of about 37°C. A suitable composition comprises from 0.1 - 21 % of the organoselenium compound(s), for example or n-hexyl selenol.

When applied in the form of sprays, solutions of the organoselenium compounds in physiologically acceptable inert carriers such as n-decane or light petrolatum and a propellant such as compressed air, nitrogen or CO₂ may be used.

When used in the form of an adhesive tape, bandage or a transdermal patch for slow release in the topical treatment of cutaneous and subcutaneous cancerous and precancerous conditions the organoselenium compound(s) may be dispersed in a plastic substrate with or without additional carriers. Further, the organoselenium compounds may be dissolved in suitable polymeric carriers for this purpose.

Cancerous lesions are typically treated by direct application of the organoselenium compound, e.g. dialkyl diselenide, containing lotion. Adhesive patches impregnated with the lotions can be used, allowing the organoselenium compounds to be slowly released into the tumor. The organoselenium compounds may also be applied in the form of creams, oils or sprays. These may contain from 0.01 - 40% by weight of the selenium compounds. Creams may contain the commonly used base materials, including sesame oil, safflower oil, wheat germ oil, stearic acid, cetyl alcohol, glycerol, triethanol amine, retinyl palmitate, allantoin, methyl paraben, imidazolidinyl urea, fragrant oils and water. Sprays contain the selenium compounds in an inert solvent such as n-decane, or light petrolatum and a propellant such as compressed air, nitrogen, or carbon dioxide.

Lotions, oils, creams or patches containing the organoselenium compounds may be applied onto entire organs suspected to harbor tumor cells. For example, such compositions may be periodically applied onto the breast, resulting in the temporary build-up of locally high concentrations of these organoselenium compounds, providing a novel means of breast cancer prevention.

In a further embodiment the invention provides a therapeutic pad comprising an organoselenium compound allowing the administration of the organoselenium compound under exclusion of oxygen, which avoids release of the organoselenium compound to the surrounding airspace.

Details of the invention and certain preferred embodiments thereof will be further understood upon reference to the following examples. Percentages are by volume unless otherwise indicated.

### Description of Fig. 1

**Figure 1** shows an example of an embodiment of pad for the anaerobic administration of the selenol-carrier mixture to the treatment area.
The pad is allowed to remain over the treatment area from 1-24 hours. As a rule this treatment is repeated 1 to 5 times, preferably 2 to 8 times, more preferably 3 times, on 3 consecutive days at which point regressive changes of the tumor and a red discoloration of the surrounding skin develops. The treated area may also appear warmer to the touch. The red discoloration is presumably not caused by an inflammation and usually disappears after 3-5 days, whereupon the treatment is repeated. Tumors may initially tum thinner and harder, the skin may turn black due to necrosis. Dead tumor tissue usually drops off or may be removed. Internal tumors such as tumors of the breast will soften and eventually undergo liquefactive necrosis. Very large tumors require repeated applications.

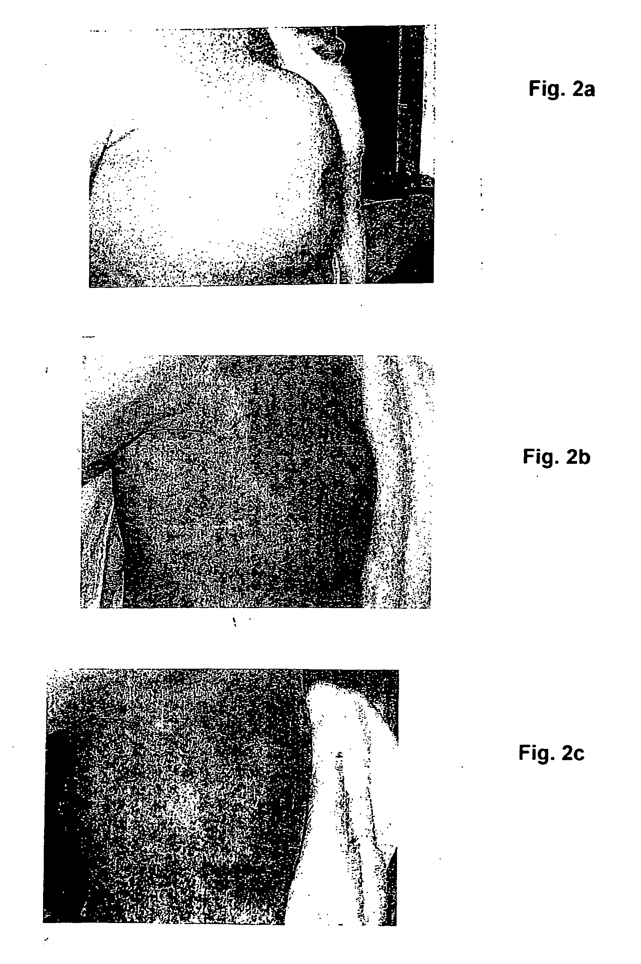

   In Fig. 1a a plug attachment (1) on the pad is shown in which an ampoule (see Fig. 1b, 2) comprising the selenol-carrier mixture is placed (see Fig. 1d). The pad comprises a gauze layer covered with removable plastic (see Fig. 1c, 4) and an adhesive (Fig. 1c. 3).
Figure 2 is a demonstration of tumor regression after n-hexyl selenol/decane transdermal monotherapy:
   a) **June 16, 2004:**
      Large, inoperable mammary tumor of a 33-year old woman
   b) **July 22, 2004:**
      Appearance after 1 month of treatment with weekly intermissions of n-hexyl selenol, 10% (wt) in 80% (v/v) n-decane, 20% light petrolatum
   c) **August 30, 2004:**
      Appearance after 2 months of therapy

### Examples

### Example 1: Female, 33 years old

Demonstration of tumor regression after n-hexyl selenol/decane transdermal monotherapy June 16, 2004:

Large, inoperable mammary tumor of a 33-year-old woman July 22, 2004:

Appearance after 1 month of treatment with weekly intermissions of n-hexyl selenol, 10% (wt) in 80% (v/v) n-decane, 20% light petrolatum.

August 30, 2004:

Appearance after 2 months of therapy

### Example 2: Male, 78 years old

Squemous cell carcinoma on right cheek, 1,5 x 1,5 cm. Treated transdermally with a 5 wt% solution of n-octylselenol in sesame oil. After 3 treatments over a period of 6 days, tumor area is softened, surrounding skin dark red. After 2 weeks, redness disappeared; no tumor visible or palpable.

### Example 3: Female, 73 years old

Newly diagnosed cancer of left breast, left upper quadrant, 2.5 x 3 x ∼ 2 cm. After 4 treatments with a 8 wt% solution of n-heptylseleonol in 80 vol. % n-decane, 20% jojoba oil, over 8 days, tumor first softened, became more movable, after 12 days tumor no longer detectable. Tumor marker ∼ 21/29 drops from 46 to 22 (measured 4 weeks after cessation of treatment).

### Example 4: Female, 70 years old

This is an example for rectal cancer:
A 70-year-old woman was diagnosed with an adenocarcinoma of the rectum, consisting of irregular mass of approximate size of 2 x 3 x 1 cm, located 8 cm from the anus. She experienced frequent bleeding, especially after defecation. The patient was treated with suppositories containing 15 wt.% of n-octylselenol in paraffin, 3 times a week for 3 weeks. After the first week, the tumor had shrunk significantly in size and had become a hard, horny mass. Bleeding began to subside. After 3 weeks the remnants of the tumor were expelled during a defecation. On digital examination no tumor mass was palpable.

### Example 5: Male, 52 years old

This is an example for bladder cancer and its prevention of recurrence.

A 52-year-old male diagnosed with an adencarcinoma of the bladder. After standard surgical treatment he developed a local recurrence 18 months later. After excision, he underwent treatment with Bcg for 6 weeks. Thereafter, he received 3 instillations of 2 ccm of a 10 wt.% solution of n-hexylselenol in n-decane in an emulsion. No recurrence after 12 months of observation.

## Claims

1. Use of
a) an organoselenium compound of the general formula
R-Se-H
in which R represents cyloalkyl, aryl, aralkyl or an alkyl group having from 1 to 50, preferably from 5 to 12 carbon atoms, wherein the terminal carbon atom of each of said alkyl groups may be substituted with a carboxyl group, or of
b) a polyselenol having 2 to 20 SeH moieties comprised in an alkyl residue having 1 to 50 carbon atoms
for the manufacture of a medicament for topical treatment and prevention of cutaneous and subcutaneous cancer and pre-cancerous conditions,
wherein the organoselenium compound is to be administered under conditions preventing its oxidation under exclusion of oxygen.

2. The use according to claim 1, wherein the organoselenium compound is n-hexyl, n-heptyl or n-octyl selenol.

3. The use according to any one of claims 1 to 2, wherein the medicament comprising a solution or dispersion of said organoselenium compound in a physiologically acceptable carrier is capable of being absorbed through skin.

4. The use according to any of claims 1 to 3, wherein the medicament comprises the organoselenium compound in an amount of from about 0.01 % to 90 % by weight, preferably 0.1 to 20% by weight.

5. The use according to any one of claims 1 to 4, wherein the pharmaceutical preparation comprises a carrier comprising an aliphatic hydrocarbon, preferably having 9 to 12 carbon atoms, the carrier being preferably selected from n-decane, n-undecane, n-dodecane or a mixture thereof.

6. The use according to any one of claims 1 to 5, wherein the pharmaceutical preparation comprises a carrier comprising at least one material selected from the group comprising sesame oil, canda oil, safflower oil, wheat germ oil, sunflower oil, avocado oil, jojoba oil, olive oil, squalane, stearic acid, cetyl alcohol, glycerol, triethanolamine, retinyl palmitate, allantoin, methyl paraben, imidazolidinyl urea, fragrant plant oils, DL-α-tocopherol, DL-α-tocopheryl acetate, vitamin A and light petrolatum.

7. The use according to any of claims 4-6, in which a therapeutic pad according to claim 8 or 9 is used for administration of the organoselenol compound under exclusion of oxygen.

8. A therapeutic pad comprising an organoselenium compound as defined in any of claims 1 to 6 allowing the administration of the organoselenium compound under exclusion of oxygen, which avoids release of the organoselenium compound to the surrounding airspace.

9. The therapeutic pad according to claim 8, which further comprises a plug attachment (1), in which an ampoule (2) comprising selenol-carrier mixture is placed and which comprises a gauze layer covered with removable plastic (4) and an adhesive (3).

## Patentansprüche

1. Verwendung von
a) einer Organoselen-Verbindung der allgemeinen Formel
R-Se-H
in welcher R Cyloalkyl, Aryl, Aralkyl oder eine Alkylgruppe mit 1 bis 50 ist, vorzugsweise von 5 bis 12 Kohlenstoffatomen, wobei das terminale Kohlenstoffatom einer jeden der besagten Alkylgruppen substituiert sein kann mit einer Carboxylgruppe, oder
b) eines Polyselenols mit 2 bis 20 SeH Gruppen, umfassend einen Alkylrest mit 1 bis 50 Kohlenstoffatomen,
für die Herstellung eines Medikaments für die topische Behandlung und Prävention von kutanem oder subkutanem Krebs oder Prä-Krebs-Bedingungen,
wobei die Organoselen-Verbindung verabreicht werden soll unter Bedingungen, die deren Oxidation unter Ausschluss von Sauerstoff verhindern.

2. Die Verwendung gemäß Anspruch 1, wobei die Organoselen-Verbindung n-Hexyl-, n-Heptyl- oder n-Octylselenol ist.

3. Die Verwendung gemäß irgendeinem der Ansprüche 1 oder 2, wobei das Medikament eine Lösung oder Dispersion von besagter Organoselen-Verbindung umfasst, in einem physiologisch verträglichen Träger, und in der Lage ist, durch die Haut absorbiert zu werden.

4. Die Verwendung gemäß irgendeinem der Ansprüche 1 bis 3, wobei das Medikament die Organoselen-Verbindung in einer Menge umfasst von ungefähr 0,01 % bis 90 Gewichts-%, vorzugsweise 0,1 bis 20 Gewichts-%.

5. Die Verwendung gemäß irgendeinem der Ansprüche 1 bis 4, wobei die pharmazeutische Zusammensetzung einen Träger umfasst, der einen aliphatischen Kohlenwasserstoff umfasst, vorzugsweise mit 9 bis 12 Kohlenstoffantomen, wobei der Träger vorzugsweise ausgewählt ist aus n-Decan, n-Undecan, n-Dodecan oder einer Mischung davon.

6. Die Verwendung gemäß irgendeinem der Ansprüche 1 bis 5, wobei die pharmazeutische Zusammensetzung einen Träger umfasst, der zumindest ein Material umfasst, das ausgewählt ist aus der Gruppe bestehend aus Sesamöl, Canolaöl, Distelöl, Weizenkeimöl, Sonnenblumenöl, Avocadoöl, Jojobaöl, Olivenöl, Squalan, Stearinsäure, Cetylalkohol, Glycerol, Trieethalonamine, Retinylpalmitat, Allantoin, Methylparaben, Imidazolidinyl-Harnstoff, Duftpflanzenöl, DL-α-tocopherol, DL-α-tocopherylacetat, Vitamin A und Leicht-Petrolatum.

7. Die Verwendung gemäß irgendeinem der Ansprüche 4 bis 6, in welchen ein therapeutisches Kissen gemäß Anspruch 8 oder 9 verwendet wird zur Verabreichung der Organoselen-Verbindung unter Ausschluss von Sauerstoff.

8. Ein therapeutisches Kissen umfassend eine Organoselen-Verbindung, wie in irgendeinem der Ansprüche 1 bis 6 definiert, welches die Verabreichung ermöglicht von Organoselen-Verbindungen unter Ausschluss von Sauerstoff, und welches die Freisetzung der Organoselen-Verbindung in den umgebenden Luftraum vermeidet.

9. Das therapeutische Kissen gemäß Anspruch 8, welches des weiteren einen aufstöpselbaren Anschluss (1) umfasst, in welchem eine Ampulle (2), die eine Selenol-Träger-Mischung umfasst, platziert wird, sowie eine Mull-Schicht umfasst, bedeckt mit entfernbarer Plastik (4), sowie einer Klebeschicht (3).

## Revendications

1. Utilisation de
a) un composé d'organosélénium de la formule générale
R-Se-H
dans lequel R représente cycloalkyle, aryle, aralkyle ou un groupe alkyle ayant de 1 à 50, de préférence de 5 à 12 atomes de carbone, où l'atome de carbone terminal de chacun desdits groupes alkyles peut être substitué par un groupe carboxyle, ou de:
b) un polysélénol ayant 2 à 20 fragments SeH compris dans un résidu alkyle ayant 1 à 50 atomes de carbone
pour la fabrication d'un médicament pour le traitement topique et la prévention du cancer cutané et sous-cutané et de conditions pré-cancéreuses,
où le composé d'organosélénium doit être administré sous des conditions empêchant son oxydation sous l'exclusion d'oxygène.

2. Utilisation selon la revendication 1, où le composé d'organosélénium est n-hexyle, n-heptyle ou n-octyle sélénol.

3. Utilisation selon l'une quelconque des revendications 1 à 2, où le médicament comprenant une solution ou dispersion dudit composé d'organosélénium dans un support physiologiquement acceptable est apte à être absorbé à travers la peau.

4. Utilisation selon l'une quelconque des revendications 1 à 3, où le médicament comprend le composé d'organosélénium en une quantité d'environ 0,01% à 90% en poids, de préférence 0,1 à 20% en poids.

5. Utilisation selon l'une quelconque des revendications 1 à 4, où la préparation pharmaceutique comprend un support comprenant une hydrocarbure aliphatique, de préférence ayant 9 à 12 atomes de carbone, le support étant de préférence sélectionné parmi n-décane, n-undécane, n-dodécane ou un mélange de ceux-ci.

6. Utilisation selon l'une quelconque des revendications 1 à 5, où la préparation pharmaceutique comprend un support comprenant au moins un matériau sélectionné dans le groupe comprenant l'huile de sésame, l'huile de canda, l'huile de carthame, l'huile de germe de blé, l'huile de tournesol, l'huile d'avocat, l'huile de jojobat, l'huile d'olive, squalane, acide stéarique, alcool cétylique, glycérol, triéthanolamine, rétinyl palmitate, allantoïne, méthyl parabène, imidazolidinyl urée, huiles de plantes de fragrance, DL-α-tocophérol, DL-α-tocophéryl acétate, vitamine A et pétrolatum léger.

7. Utilisation selon l'une quelconque des revendications 4 à 6, où une compresse thérapeutique selon la revendication 8 ou 9 est utilisé pour l'administration du composé d'organosélénol sous exclusion d'oxygène.

8. Compresse thérapeutique comprenant un composé d'organosélénium tel que défini dans l'une quelconque des revendications 1 à 6 permettant l'administration du composé d'organosélénium sous exclusion d'oxygène, qui évite la libération du composé d'organosélénium à l'espace d'air environnant.

9. Compresse thérapeutique selon la revendication 8, qui comprend en outre une fixation de bouchon (1), dans laquelle une ampoule (2) comprenant un mélange sélénium-support est placée et qui comprend une couche de gaze recouverte d'un plastique amovible (4) et d'un adhésif (3).
